(19) Europäisches Patentamt
European Patent Office
Office européen des brevets

(11) **EP 3 668 637 B1**

(12) **EUROPÄISCHE PATENTSCHRIFT**

(45) Veröffentlichungstag und Bekanntmachung des Hinweises auf die Patenterteilung:
**17.01.2024 Patentblatt 2024/03**

(21) Anmeldenummer: **18755451.4**

(22) Anmeldetag: **14.08.2018**

(51) Internationale Patentklassifikation (IPC):
**B01D 63/00** *(2006.01)* **B01D 65/00** *(2006.01)*
**A61M 1/16** *(2006.01)* **C02F 1/44** *(2023.01)*

(52) Gemeinsame Patentklassifikation (CPC):
**B01D 65/00; A61M 1/1652; B01D 63/00;**
A61M 2209/08; B01D 2313/06; B01D 2313/105;
B01D 2313/125; B01D 2317/04; C02F 1/44;
C02F 2201/004

(86) Internationale Anmeldenummer:
**PCT/EP2018/071989**

(87) Internationale Veröffentlichungsnummer:
**WO 2019/034637 (21.02.2019 Gazette 2019/08)**

(54) **ANSCHLUSSVORRICHTUNG FÜR EIN ROHRFÖRMIGES FILTERMODUL**

CONNECTION DEVICE FOR A TUBULAR FILTER MODULE

DISPOSITIF DE RACCORDEMENT CONÇU POUR UN MODULE DE FILTRAGE TUBULAIRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **16.08.2017 DE 102017214263**

(43) Veröffentlichungstag der Anmeldung:
**24.06.2020 Patentblatt 2020/26**

(73) Patentinhaber: **Fresenius Medical Care Deutschland GmbH**
**61352 Bad Homburg (DE)**

(72) Erfinder:
• **HEINZ, Thomas**
**66606 St. Wendel (DE)**
• **KELLER, Torsten**
**66606 St. Wendel (DE)**
• **WIESEN, Gerhard**
**61352 Bad Homburg (DE)**

(74) Vertreter: **Ricker, Mathias**
**Wallinger Ricker Schlotter Tostmann**
**Patent- und Rechtsanwälte Partnerschaft mbB**
**Zweibrückenstraße 5-7**
**80331 München (DE)**

(56) Entgegenhaltungen:
EP-A1- 2 260 927      EP-A2- 0 408 375
EP-A2- 1 775 538      WO-A1-01/64312
WO-A1-2013/048801     WO-A1-2016/185506
DE-C1- 19 925 297     JP-A- 2000 325 712
US-A1- 2015 001 140   US-B1- 6 220 557

## Beschreibung

### THEMA DER ERFINDUNG

Die Erfindung betrifft eine Anschlussvorrichtung zur Aufnahme von Filtermodulen, insbesondere rohrförmigen Filtermodulen. Weiterhin betrifft die Erfindung ein Filtrationssystem, aufweisend eine Anschlussvorrichtung und ein Filtermodul, sowie eine Baugruppe bestehend aus einer Vielzahl der Filtrationssysteme.

### HINTERGRUND DER ERFINDUNG

Filtermodule, insbesondere rohrförmige Filtermodule, werden in der Wasseraufbereitung und in der Dialyse von nierenkranken Patienten verwendet. Die rohrförmigen Filtermodule zeichnen sich durch ein längliches, insbesondere zylindrisches Gehäuse aus, und können als Rohre bezeichnet werden. Die Enden der Rohre sind durch entsprechende Kappen verschlossen. Solche rohrförmigen Filtermodule sind im Allgemeinen so ausgestaltet, dass der Raum im Inneren des rohrförmigen Gehäuses durch eine Membranwand in wenigstens zwei Strömungsräume unterteilt ist, insbesondere kann der Raum im Inneren des rohrförmigen Gehäuses auch durch eine Vielzahl von Membranwänden in wenigstens zwei Strömungsräume unterteilt sein. Diesbezüglich werden z.B. Hohlfasermembranbündel verwendet, um einen ersten und einen zweiten Strömungsraum in dem rohrförmigen Gehäuse zu trennen. Der erste Strömungsraum kann dabei den Raum zwischen den Hohlfasermembranen im Inneren des Gehäuses einnehmen, und die Lumenseiten der Hohlfasermembranen bilden den zweiten Strömungsraum. Die Hohlfasermembranen sind in rohrförmigen Filtermodulen an den Enden der Hohlfasermembranen und im Bereich des Endes des rohrförmigen Gehäuses des Filtermoduls im Regelfall in einer Dichtungsmasse vergossen, so dass der erste Strömungsraum und der zweite Strömungsraum nur über die Membranwandungen hinweg in Fluidverbindung stehen können. Solche Filtermodule, sind im Bereich der Wasseraufbereitung und der Dialyse von nierenkranken Patienten hinlänglich bekannt und in Verwendung.

Ein rohrförmiges Filtermodul weist wenigstens zwei Fluidzugänge auf, wobei jeder Strömungsraum mit wenigstens einem Fluidzugang verbunden ist. Das hinzufließende Fluid, das in den ersten Strömungsraum eintritt, wird dabei auch als "Feed" bezeichnet. Das Feed-Fluid wird über die Membrantrennwand filtriert und tritt als filtriertes Fluid in den zweiten Strömungsraum über, wo es durch den zweiten Fluidzugang abgeleitet werden kann. Die filtrierte Flüssigkeit wird dabei auch als "Filtrat" bezeichnet.

Weiterhin ist im Stand der Technik bekannt, dass solche Module auch einen dritten Fluidzugang aufweisen können. Dieser kann am Filtermodul so angeordnet sein, dass er in Fluidverbindung mit dem ersten Strömungsraum, der Feed-Seite, oder dem zweiten Strömungsraum, der Filtratseite, in Fluidverbindung steht. Dieser dritte Anschluss kann z.B. dazu dienen, Feed oder Filtrat abzuleiten oder zurückzuführen. Der dritte Anschluss kann auch dazu vorgesehen sein, eine Rückspülung einer kontaminierten Seite der Membran zu ermöglichen. Die Anschlüsse dieser rohrförmigen Filtermodule können z.B. über Schläuche konnektiert sein und mit einer Fluidverarbeitungsmaschine, wie z.B. einer Dialysemaschine oder einer Wasseraufbereitungsanlage verbunden sein. Bei großen Filtrationsanlagen, wie sie z.B. in der Wasseraufbereitung bekannt sind, werden die Anschlüsse fest mit Anschlussrohren verbunden. Bekannt sind rohrförmige Filtermodule mit Fluidanschlüssen, die an den jeweiligen Enden des rohrförmigen Modulgehäuses angebracht sind. Die Anordnung der Fluidanschlüsse an den Enden der rohrfömigen Gehäuse der Filtermodule erfolgt dabei, um eine lokale Trennung zwischen Feedstrom und Filtrat-Seite zu erreichen. Dadurch soll eine Kreuzkontamination der Filtratseite durch die Feedseite vermieden werden. In einer aufrechten Position des rohrförmigen Filtermoduls befindet sich der Filtratanschluss meistens oben, der Feedanschluss ist unten, ein dritter Anschluss ist seitlich am Filter angeordnet, über den Retentat aus dem Filtermodul abgeleitet wird.

Im Bereich von Trinkwasseraufbereitungsanlagen ist es bekannt, eine Vielzahl von rohrförmigen Filtermodulen über die an den Enden der Filtermodule angebrachten Fluidzugänge durch Verbindungsrohre zu einer Baugruppe zu verbinden. Zum einen kann über eine derartige Rohrverbindung der Filtermodule untereinander eine Filtrationskaskade über mehrere Filtermodule innerhalb einer Baugruppe hinweg erzeugt werden. Weiterhin besitzt eine derartige Baugruppe durch die parallel angeordneten rohrförmigen Filtermodule und die Querverbindung der Verbindungsrohre eine selbsttragende Struktur, die dieser Anordnung Stabilität und Kompaktheit verleiht.

Die konstruktive Kompaktheit solcher Baugruppen in Filtrationsanlagen macht es jedoch schwer, auftretende Defekte zu beheben, insbesondere ist der Austausch von einzelnen Filtermodulen in einer Baugruppe mit großem Aufwand verbunden. Daher wird bei auftretenden Defekten mitunter die gesamte Baugruppe ausgetauscht, um den Reparaturaufwand zu minimieren. Allerdings ist diese Vorgehensweise kostenintensiv und bei kleineren Filtrationsanlagen und für Privatanwender wirtschaftlich nachteilig.

Weiterhin sind die beschriebenen Baugruppen in Filtrationsprozessen, die Temperaturschwankungen unterworfen sind, auch von einer wärmebedingten Materialausdehnung betroffen, d.h., dass sich insbesondere die Längsausdehnung der rohrförmigen Filtermodule und der Verbindungsrohre je nach Temperaturwechsel der Feed- und Filtratfluide bemerkbar macht. Die Längsausdehnung der rohrförmigen Filtermodule muss dabei von den querverbindenden Verbindungsrohren aufgenommen werden, was insgesamt zu Materialspannungen und insbesondere auch zu konstruktiven Spannungen innerhalb der Filtermodul-Baugruppe führt. Solche Spannungen sind als nachteilig zu beurteilen, da sie zu Defekten oder zu Undichtigkeiten im fluidführenden System der Baugruppe führen können.

Die WO 2012/113505 A1 beschreibt eine Vorrichtung zur Aufbereitung von Wasser für die Anwendungen in der häuslichen Wasserversorgung. Die Vorrichtung umfasst einen Filterkopf, einen Einlass und einen Auslass, eine Filtertasse und ein Filterelement, das in die Filtertasse eingebracht werden kann. Mittels einer Betätigungseinrichtung wird die Filtertasse von einer Entnahmeposition in eine Verschlussposition gebracht und umgekehrt. In der Entnahmeposition ist das Filterelement aus der Filtertasse entnehmbar bzw. kann ein Filterelement in die Filtertasse eingebracht werden. In der Verschlussposition wird die Filtertasse an den Filterkopf gedrückt, so dass die Filtertasse unter Vorspannung steht und das Filterelement in der Filtertasse abdichtend mit dem Einlass und dem Auslass der Vorrichtung in Verbindung steht.

Die DE 197 27 251 A1 zeigt eine Filtervorrichtung und ein Halterungssystem, das einen einfachen und sicheren Anschluss an ein Schlauch- oder Rohrleitungssystem ermöglichen soll. Die Filtervorrichtung weist dabei wenigstens zwei Anschlüsse auf, insbesondere vier Anschlüsse, die auf einer Seite des Gehäuses der Filtervorrichtung angeordnet sind, so dass deren Mittellinien parallel angeordnet sind. Die Filtervorrichtung wird in einer Wasseraufbereitungseinheit zur Filtration von Wasser verwendet, das für die Dialysetherapie von nierenkranken Menschen verwendet wird. Die Halterungsvorrichtung weist Führungen auf, die mit Gegenführungsstücken an der Filtervorrichtung eingreifen können. Weiterhin wird eine Fluidverbindung der Filtervorrichtung durch ausfahrbare Tüllen der Haltevorrichtung hergestellt. Die Tüllen können dabei durch eine Hebelvorrichtung in die Anschlüsse der Filtervorrichtung eingeschoben werden.

Die US 2015/0001140 beschreibt ein Filtrationssystem für die Wasseraufbereitung mit einer schwenkbaren Abdeckung, einer Halterungseinrichtung und einer Filterkartusche. Die Filterkartusche besteht dabei aus einem Gehäuse mit zwei seitlich und horizontal am Gehäuse angeordneten Anschlüssen. Die Halterungseinrichtung weist zwei zu den Anschlüssen des Filters komplementäre Aufnahmen auf, um eine Fluidverbindung zu der Filterkartusche herzustellen. Die schwenkbare Abdeckung dient dabei als Verriegelungsvorrichtung und sichert die Position der Anschlüsse in den komplementären Aufnahmen.

Die JP2000-325712 offenbart ein Filtermodul mit zwei radialen Anschlüssen, sowie eine Anschlussvorrichtung zur Aufnahme des Filtermoduls.

Die im Stand der Technik bekannten Filtrationssysteme sind hinsichtlich einer einfachen Handhabbarkeit und eines wartungsarmen Betriebs nicht zufriedenstellend. Das Problem der Kompensation der thermischen Materialausdehnung wird durch die bekannten Systeme nicht zufriedenstellend gelöst. Darüber hinaus besteht weiterhin das Bedürfnis, ein System bereitzustellen, das eine Baugruppe aus einer Vielzahl von Filtervorrichtungen darstellt, die bezüglich der thermischen Materialausdehnung optimiert sind, andererseits aber auch den Austausch einzelner Filtermodule innerhalb der Baugruppe mit geringem Aufwand ermöglicht.

## AUFGABE DER ERFINDUNG

In einem ersten Aspekt der Erfindung bestand daher die Aufgabe, eine Anschlussvorrichtung für ein Filtermodul, insbesondere ein rohrförmiges Filtermodul, bereitzustellen, mit der eine Fuidverbindung zu dem Filtermodul herstellbar ist und die einen einfachen Austausch eines Filtermoduls ermöglicht, wobei die Anschlussvorrichtung so ausgestaltet ist, dass eine thermische Materialausdehnung nicht zu schädlichen Materialspannungen führt.

In einem weiteren Aspekt wird ein Filtermodul beschrieben, insbesondere ein rohrförmiges Filtermodul, das in einer Anschlussvorrichtung gemäß dem ersten Aspekt einfach einzubauen oder austauschbar ist, wobei das Filtermodul so ausgestaltet ist, dass im Zustand der Fluidverbindung mit der Anschlussvorrichtung eine thermische Materialausdehnung ohne den Aufbau von Materialspannungen möglich ist.

In einem weiteren Aspekt der Erfindung bestand die Aufgabe, ein Filtrationssystem aus einer Anschlussvorrichtung und einem Filtermodul, insbesondere einem rohrförmigen Filtermodul, bereitzustellen, das eine Wärmeausdehnung des Filtermoduls in der Anschlussvorrichtung zulässt und durch welches Materialspannungen in dem Filtrationssystem vermieden werden.

Eine weitere Aufgabe der Erfindung bestand darin, eine Baugruppe aus einer Vielzahl von Filtrationssystemen für Fluidaufbereitungsanlagen, insbesondere Wasseraufbereitungsanlagen, bereitzustellen, in denen rohrförmige Filtermodule, insbesondere rohrförmige Filtermodule, durch Anschlussvorrichtungen so aufgenommen werden, dass eine Wärmeausdehnung der Filtermodule ermöglicht wird, ohne dass konstruktive Materialspannungen auftreten.

## ZUSAMMENFASSUNG

Erfindungsgemäß wird in einem ersten Aspekt der Erfindung die Aufgabe durch eine Anschlussvorrichtung nach Anspruch 1 gelöst. Die Unteransprüche 2 bis 7 stellen bevorzugte Ausführungsformen des ersten Aspekts der Erfindung dar.

In einem zweiten Aspekt wird ein Filtermodul beschrieben.

In einem dritten Aspekt wird die Aufgabe durch ein Filtrationssystem nach Anspruch 8 gelöst.

In einem vierten Aspekt der Erfindung wird die Aufgabe durch eine Baugruppe nach Anspruch 9 gelöst.

In einem fünften Aspekt wird die Verwendung eines Filtermoduls beschrieben.

In einem sechsten Aspekt der Erfindung wird die Aufgabe durch die Verwendung einer Anschlussvorrichtung nach wenigstens einem der Ansprüche 1 bis 7 in einem Prozess nach Anspruch 10 gelöst.

## DETAILLIERTE BESCHREIBUNG DER ERFINDUNG

Ein erster Aspekt der Erfindung betrifft eine Anschlussvorrichtung zur Aufnahme mindestens eines Filtermoduls, das durch Bewegung entlang einer Richtung (E) aus einer proximalen Position in eine distale Aufnahmeposition an die Anschlussvorrichtung anschließbar ist, insbesondere eines rohrförmigen Filtermoduls, mit wenigstens einem seitlichen Anschluss, insbesondere einem radialen Anschluss, aufweisend zumindest eine erste Verbindungseinrichtung mit einem ein inneres Lumen aufweisenden Konnektorstück, welches ein in der Aufnahmeposition zum Filtermodul weisendes, proximales Ende und ein zur Anschlussvorrichtung weisendes distales Ende aufweist, wobei das proximale Ende des Konnektorstücks ausgebildet ist, eine Konnektion des Konnektorstücks an dem seitlichen, insbesondere an dem radialen Anschluss des Filtermoduls zu ermöglichen, und mit zumindest einem ersten und einem zweiten Führungsteil mit jeweils einem proximalen Ende und einem distalen Ende, die einen Aufnahmebereich der Verbindungseinrichtung zur Aufnahme eines Filtermoduls, insbesondere eines rohrförmigen Filtermoduls definieren, wobei zumindest das erste Führungsteil eine zu dem Aufnahmebereich gerichtete Seite mit zumindest einer Führungsfläche aufweist, die vom proximalen Ende zum distalen Ende des ersten Führungsteils verläuft und am proximalen Ende des Konnektorstücks anschließt, wobei die zum Aufnahmebereich gerichtete, die Führungsfläche aufweisende Seite des Führungsteils, insbesondere in einem Querschnitt, s-förmig ausgestaltet ist, wobei die S-Form der Führungsfläche durch zwei aneinanderreihende Krümmungsabschnitte (R1, R2) entsteht und der eine Querschnitt dabei mit einer Fläche zusammenfällt, die durch den Aufnahmebereich (205) und die beiden Führungsteile (202) der Verbindungseinrichtung verläuft.

Die Anschlussvorrichtung weist den Vorteil auf, dass ein Filtermodul, insbesondere ein rohrförmiges Filtermodul, in die Verbindungseinrichtung eingeführt werden kann, ohne dass ein Verkanten des seitlichen, insbesondere radialen Anschlusses erfolgt. Insbesondere kann das Filtermodul mit dem seitlichen, insbesondere radialen Anschluss in den Aufnahmebereich der Verbindungseinrichtung eingebracht werden, so dass lediglich der Anschluss in Richtung des Konnektorstücks ungefähr, aber nicht zielgerichtet ausgerichtet sein muss. Beim weiteren Einschieben des rohrförmigen Filtermoduls in den Aufnahmebereich der Verbindungseinrichtung entlang einer Richtung E aus einer proximalen Position in eine distale Aufnahmeposition wird der seitliche, insbesondere radiale Anschluss des rohrförmigen Filtermoduls durch das wenigstens erste Führungsteil ausgerichtet. Bei diesem Vorgang des Ausrichtens wird das Filtermodul, insbesondere das rohrförmige Filtermodul, um seine Längsachse gedreht. Die Ausrichtung erfolgt dabei dadurch, dass der seitliche, insbesondere der radiale Anschluss des rohrförmigen Filtermoduls die seitlichen Führungsflächen der Führungsteile berührt, die zu dem Aufnahmebereich der Verbindungsrichtung ausgerichtet sind. Durch den Vorgang des Einbringens des rohrförmigen Filtermoduls in den Aufnahmebereich der Verbindungseinrichtung wird der Anschluss entlang der s-förmigen Führungsflächen des ersten Führungsteils so ausgerichtet, dass die Mittelachse des Anschlusses mit der Achse des Konnektorstücks zusammenfällt. Dadurch ist das rohrförmige Filtermodul so ausgerichtet, dass eine Fluidverbindung zwischen dem Anschluss und dem Konnektorstück hergestellt werden kann.

Der Begriff "*Anschlussvorrichtung*" bezeichnet dabei eine konstruktive Einheit, die dazu vorbereitet ist, eine Fluidverbindung mit einem Filtermodul herzustellen. Eine Anschlussvorrichtung im Sinne der vorliegenden Anmeldung weist wenigstens eine oder mehrere Funktionselemente auf, wobei eine Verbindungseinrichtung wenigstens eine dieser Funktionselemente ist. Die Anschlussvorrichtung kann auch mehrere Verbindungseinrichtungen aufweisen, mit denen eine Fluidverbindung mit wenigstens einem Anschluss des Filtermoduls herstellbar ist, um Fluide zum rohrförmigen Filtermodul hinzuleiten oder von dem Filtermodul abzuleiten. Weiterhin kann eine Anschlussvorrichtung auch konstruktive Teile zur mechanischen Halterung des Filtermoduls aufweisen. Weiterhin können auch Verriegelungseinrichtungen vorhanden sein, mit denen das Filtermodul in der Anschlussvorrichtung fixiert werden kann, oder mit denen die Fluidverbindung zwischen Anschluss- und Verbindungseinrichtung gesichert werden kann.

Der Begriff "*rohrförmiges Filtermodul*" bezeichnet ein Filtermodul, dessen Gehäuse als Rohr bezeichnet werden kann. Unter dem Begriff "*Rohr*" werden solche Gehäuse verstanden, die einen kreisrunden, ovalen oder eckigen Querschnitt aufweisen. Für die Aufnahme des Filtermoduls in den Aufnahmebereich der Verbindungseinrichtung ist entscheidend, dass das Filtermodul in einem Abschnitt des seitlichen, insbesondere des radialen Anschlusses einen Querschnitt aufweist, der mit dem Aufnahmebereich der Verbindungseinrichtung zusammenwirken kann. Bevorzugt weist das rohrförmige Filtermodul ein zylindrisches Gehäuse, also ein Gehäuse mit einem kreisrunden Querschnitt, auf. Es können aber auch rohrförmige Filtermodule bevorzugt sein, die ein ovales oder eckiges Gehäuse aufweisen, solange im Bereich des seitlichen Anschlusses ein Abschnitt des Gehäuses mit kreisrundem Querschnitt ausgearbeitet ist. Solche Filtermodule sind insbesondere als Hohlfasermembranfilter im Stand der Technik bekannt. Bei Hohlfasermembranfiltern ist eine Vielzahl von Hohlfasermembranen in einem rohrförmigen zylindrischen Gehäuse eines Filtermoduls angeordnet. Die Hohlfasermembranen unterteilen dabei den inneren Raum des Gehäuses in wenigstens zwei Strömungsräume, die über Endkappen, die an den Enden des rohrförmigen Gehäuses angebracht sind und über jeweilige Anschlüsse mit Fluiden angeströmt werden können. Zumindest zwei Anschlüsse dienen dazu, ein Feed-Fluid und ein Filtrat-Fluid zum Filtermodul hin- und wieder abzuleiten. Unter "rohrförmig" wird dabei weiter verstanden, dass die Längsausdehnung des Gehäuses größer ist als der Durchmesser des Querschnitts des Filtermoduls.

Im Sinne der vorliegenden Anmeldung bezeichnet der Begriff "Verbindungseinrichtung" eine Einrichtung, mit der eine Fluidverbindung mit den Anschlüssen des Filters herstellbar ist. Die Verbindungseinrichtung weist mindestens ein Konnektorstück auf, welches mit dem Anschluss des Filtermoduls verbindbar ist. Das "Konnektorstück" besteht dabei aus einem Schlauch oder einer Hülse mit einem proximalen und einem distalen Ende, wobei das proximale Ende dafür vorgesehen ist, eine Konnektion, d.h. eine Verbindung, insbesondere fluiddichte Verbindung, mit dem Anschluss des Filtermoduls herzustellen. Schlauch oder Hülse weisen einen inneren Hohlraum auf, durch den entsprechende Fluide zum Filtermodul hingeführt oder abgeführt werden können. Der Hohlraum wird als "Lumen" bezeichnet und erstreckt sich von dem proximalen Ende bis zum distalen Ende des Konnektorstücks.

Der Begriff "Anschluss" bezeichnet dabei einen Zugang zum rohrförmigen Filtermodul. Der Anschluss kann in Form einer Hülse ausgearbeitet und an dem Gehäuse oder an den Endkappen des rohrförmigen Filtermoduls angebracht sein. Der Anschluss weist zumindest eine Öffnung auf, durch die Fluide in den ersten oder zweiten Strömungsraum des rohrförmigen Filtermoduls eingeleitet oder aus diesen Strömungsräumen abgeleitet werden können. Vorzugsweise ist der Anschluss zylindrisch ausgearbeitet und weist ein inneres Lumen auf.

Das Konnektorstück kann vorzugsweise aus einem biegesteifen Kunststoffmaterial oder aus einem Metall bestehen. Dabei kann das Konnektorstück so ausgearbeitet sein, dass das Lumen zumindest in einem Endabschnitt des Konnektorstücks gegenüber den äußeren Abmessungen des Anschlusses des Filtermoduls ein Übermaß aufweist, so dass eine Konnektion durch Einschieben des Anschlusses in das Konnektorstück möglich ist. Vorzugsweise sind Konnektorstück und Anschluss zylindrisch ausgearbeitet. In einer Alternative kann das Konnektorstück aber auch so ausgearbeitet sein, dass das Lumen des Anschlusses des Filtermoduls gegenüber den äußeren Abmessungen des Konnektorstücks ein Übermaß aufweist, so dass das Konnektorstück in das Ende des Anschlusses einschiebbar ist und eine Konnektion erfolgen kann. Eine Dichtung der Konnektion kann durch Dichtelemente ausgeführt werden, die im Bereich der Kontaktflächen von Konnektorstück und Anschluss eingebracht sind. Solche Dichtelemente sind z.B. O-Ringe, die umfänglich auf der äußeren Oberfläche oder auf der inneren Oberfläche, lumenseitig des Anschlusses des Filtermoduls aufgebracht sind. Alternativ oder zusätzlich können solche O-Ringe auch auf den Konnektorstücken auf der äußeren oder der inneren, lumenseitigen Oberfläche der Konnektorstücke aufgebracht sein.

Zusätzlich können die Enden des Konnektorstücks und des Anschlusses durch durchstoßbare Septen, vorzugsweise Schlitzsepten, abgedeckt sein, um eine Kontamination des Anschlusses oder des Konnektorstücks zu vermeiden. Im Sinne der vorliegenden Anmeldung können Konnektorstück und der Anschluss des rohrförmigen Filtermoduls trotz der Abdeckung durch die Septen konnektiert werden. Der Begriff "Öffnung" bezeichnet daher im Sinne der vorliegenden Anmeldung auch solche Öffnungen, die durch durchstoßbare Septen abgedeckt sind. Entsprechende Schlitzsepten bestehen vorzugsweise aus einem elastischen Kunststoffmaterial, insbesondere aus einem thermoplastischen elastomeren Material.

Die Verbindungseinrichtung weist weiterhin ein erstes und ein zweites Führungsteil mit einem proximalen Ende und einem distalen Ende auf, wobei die Führungsteile so an der Verbindungseinrichtung angebracht sind, dass ein Aufnahmebereich zwischen den Führungsteilen entsteht. Der Aufnahmebereich ist dafür vorgesehen, dass ein Filtermodul, insbesondere rohrförmiges Filtermodul in die Verbindungseinrichtung eingebracht werden kann und dabei in dem Aufnahmebereich aufgenommen wird. Insbesondere wird das Filtermodul, insbesondere das rohrförmige Filtermodul, durch die zumindest beiden Führungsteile in dem Aufnahmebereich gehalten. In einer bevorzugten Ausführung ist entsprechend der rohrförmigen Geometrie des Filtermoduls der Aufnahmebereich der Verbindungseinrichtung, der durch die Führungsteile definiert wird, in einem Teil kreissegmentförmig so definiert, dass das in die Verbindungseinrichtung eingebrachte rohrförmige Filtermodul von den Führungsteilen im Aufnahmebereich umschlossen wird. Das bedeutet, dass die Führungsteile in einem Bereich, der zum Aufnahmebereich hingerichtet ist, eine geschwungene Form, insbesondere eine kreissegmentförmige Form, besitzen, die sich an das Gehäuse des rohrförmigen Filtermoduls anlegen kann.

Die Führungsteile weisen weiterhin zumindest eine oder mehrere zu dem Aufnahmebereich der Verbindungseinrichtung ausgerichtete Führungsflächen auf, die so ausgeformt sind, dass sie einerseits das Filtermodul, insbesondere rohrförmige Filtermodul, in dem Aufnahmebereich umschließen können, andererseits aber auch so ausgelegt sind, dass das Filtermodul beim Einbringen in den Aufnahmebereich der Verbindungseinrichtung geführt und ausgerichtet wird. Insbesondere sind die Führungsflächen der Führungsteile dafür vorgesehen, den Anschluss des rohrförmigen Filtermoduls beim Einbringen in den Aufnahmebereich der Verbindungseinrichtung so auszurichten, dass der Anschluss in Richtung des Konnektorstücks gelenkt wird. Das Filtermodul, insbesondere das rohrförmige Filtermodul, wird dabei beim Ausrichten und Einbringen in den Aufnahmebereich um seine Längsachse rotiert. Die Führungsflächen der Führungsteile sind daher in weiteren Abschnitten gekrümmt, insbesondere kreissegmentförmig ausgebildet. Die Führungsflächen der Führungsteile sind so ausgebildet, dass die gekrümmten Abschnitte, insbesondere die kreissegmentförmigen Bereiche, von dem proximalen Ende bis zum distalen Ende aneinander anschließen und insbesondere in einem Querschnitt eine S-Form der Führungsfläche ergeben, die an das proximale Ende des Konnektorstücks anschließt. Der hier bezeichnete "*Querschnitt*" fällt dabei insbesondere mit einer Fläche zusammen, die durch den Aufnahmebereich und die beiden Führungsteile der Verbindungseinrichtung verläuft und innerhalb der die Richtung (E) verlaufen kann. Die Radien der Krümmungsabschnitte, insbesondere der kreissegmentförmigen Bereiche, sind in Abhängigkeit vom Durchmesser des Filtermoduls, insbesondere des rohrförmigen Filtermoduls, dabei so aufeinander abgestimmt, dass die Ausrichtung des Anschlusses beim Einbringen des Filtermoduls in den Aufnahmebereich der Verbindungseinrichtung erfolgen kann. Insbesondere ist der Radius des ersten kreissegmentförmigen Krümmungsabschnitts, der am proximalen Ende des Konnektorstücks anschließt, kleiner gewählt als der Radius des zweiten kreissegmentförmigen Krümmungsabschnitts, der an den ersten kreissegmentförmigen Krümmungsabschnitt anschließt. Weiter ist insbesondere der Radius des zweiten kreissegmentförmigen Krümmungsabschnitts gleich groß oder größer als der Radius des Filtermoduls, insbesondere des rohrförmigen Filtermoduls. Insbesondere ist der Radius des ersten kreissegmentförmigen Krümmungsabschnitts weniger als halb so groß wie der Radius des zweiten kreissegmentförmigen Krümmungsabschnitts, oder in alternativen Ausführungsformen ist der Radius des ersten kreissegmentförmigen Krümmungsabschnitts ungefähr um mehr als den Faktor 0,3 kleiner als der Radius am zweiten kreissegmentförmigen Krümmungsabschnitt.

Gemäß einer weiteren Ausführungsform sind die s-förmigen Führungsflächen der Führungsteile so ausgebildet, dass an dem ersten kreissegmentförmigen Krümmungsabschnitt, der am proximalen Ende des Konnektorstücks anschließt, ein linearer Abschnitt vorgesehen ist, an den wiederum der zweite kreissegmentförmige Krümmungsabschnitt anschließt.

In einer weiteren Ausführungsform sind die Kreissegmente der Krümmungsabschnitte so ausgebildet, dass die Winkel der Kreissegmente folgende Beziehung erfüllen:

$$\tan \alpha < \frac{1}{\mu} \qquad \text{Gleichung 1}$$

wobei

$\alpha$ der Winkel des Kreissegments ist und
$\mu$ die Haftzahl der Materialpaare ist.

Das Materialpaar wird dabei aus dem seitlichen, insbesondere radialem Anschluss des Filtermoduls und der, oder den Führungsflächen der Führungsteile der Verbindungseinrichtung gebildet. Die Haftzahl $\mu$ ist eine Größe, die dem Fachmann bekannt ist. Eine Definition findet sich z.B. in: "Dubbel, Taschenbuch für den Maschinenbau, Auflage 22, Springer Verlag 2007." Die Einhaltung der oben gezeigten Gleichung 1 ermöglicht ein besonders reibungsarmes Einbringen des Filtermoduls in den Aufnahmebereich der Verbindungseinrichtung.

Der Anschluss des rohrförmigen Filtermoduls wird dadurch beim Einbringen in den Aufnahmebereich der Verbindungseinrichtung von einer nicht ausgerichteten Position durch die seitliche Führungsfläche gelenkt. Der Anschluss wird dabei an der in einem Querschnitt s-förmigen Führungsfläche des Führungsteils entlanggeführt und so ausgerichtet, dass in einer ausgerichteten Position die Öffnung des Anschlusses des Filtermoduls der Öffnung am proximalen Ende des Konnektorstücks der Verbindungseinrichtung gegenübersteht und durch weiteres Einschieben des rohrförmigen Filtermoduls zur Konnektion gebracht werden kann.

Durch die Konnektion wird der Anschluss des Filtermoduls, insbesondere des rohrförmigen Filtermoduls, in Fluidverbindung mit der Anschlussvorrichtung gebracht. Vorzugsweise sind Konnektorstück und Anschluss in überlappendem Eingriff, d.h. dass das Konnektorstück in den Anschluss des Filtermoduls, insbesondere rohrförmigen Filtermoduls, bei der Konnektion eingeschoben ist, oder, dass der Anschluss des Filtermoduls, insbesondere rohrförmigen Filtermoduls, in das Lumen des Konnektorstücks eingeschoben ist. Die Konnektion über Konnektorstück und Anschluss kann arretiert werden und dient somit gleichzeitig als Fixierung für das rohrförmige Filtermodul in der Anschlussvorrichtung. Dadurch ergibt sich der Vorteil, dass das Filtermodul, insbesondere das rohrförmige Filtermodul, am seitlichen, insbesondere radialen Anschluss über die Verbindungseinrichtung konnektiert und fixiert wird. Da die Fixierung über die Konnektion des radialen Anschlusses erfolgt, kann sich das Filtermodul, insbesondere das rohrförmige Filtermodul in Längsausrichtung durch Wärmeausdehnung ausdehnen oder zusammenziehen, ohne dass es zu nachteiligen Materialspannungen kommt.

In einer weiteren Ausführungsform weist das zweite Führungsteil ebenfalls eine zu dem Aufnahmebereich der Verbindungseinrichtung gerichtete und im Querschnitt s-förmige Seite mit einer Führungsfläche auf, die am proximalen Ende des Konnektorstücks anschließt, wobei die S-Form durch zwei aneinanderreihende Krümmungsabschnitte entsteht und der eine Querschnitt dabei mit einer Fläche zusammenfällt, die durch den Aufnahmebereich und die beiden Führungsteile der Verbindungseinrichtung verläuft. Die Handhabung wird dadurch vereinfacht, dass der Anschluss des Filtermoduls, insbesondere des rohrförmigen Filtermoduls, über beide Führungsflächen beim Einführen des rohrförmigen Filtermoduls in den Aufnahmebereich der Verbindungseinrichtung in Richtung des Konnektorstücks geführt werden kann. Dadurch können die Öffnungen des Anschlusses und des Konnektorstücks am proximalen Ende des Konnektorstücks entweder über die erste oder die zweite Führungsfläche der Führungsteile in eine gegenüberliegende Position gebracht werden und so leichter und schneller konnektiert werden.

In einer weiteren Ausführungsform sind das erste Führungsteil und das zweite Führungsteil auskragend am Konnektorstück, vorzugsweise am proximalen Ende des Konnektorstücks, angebracht. Eine derartige Ausgestaltung lässt eine integrale Bauweise der Verbindungseinrichtung zu. Insbesondere kann die Verbindungseinrichtung als einstückiges Bauteil in einem einstufigen Spritzgussprozess aus Kunststoff hergestellt werden.

In einer weiteren Ausführungsform weist die zum Aufnahmebereich des Filtermoduls, insbesondere rohrförmigen Filtermoduls, gerichtete s-förmige Seite der Verbindungseinrichtung des ersten Führungsteils oder des ersten Führungsteils und des zweiten Führungsteils eine obere und eine untere Führungsfläche auf, so dass zwischen der oder den oberen und der oder den unteren Führungsflächen eine Nut angeordnet ist. Relativ zur Nut verlaufen die Führungsflächen dabei auf einem oberen und einem unteren Vorsprung. Die so ausgebildete Nut unterstützt die Führung des Filtermoduls, insbesondere des rohrförmigen Filtermoduls, beim Einbringen in den Aufnahmebereich der Verbindungseinrichtung. Es ist vorgesehen, dass das Filtermodul, insbesondere das rohrförmige Filtermodul, das mit der Verbindungseinrichtung zur Konnektion gebracht werden soll, eine zur Nut komplementäre umfängliche Auflagefläche oder mehrere umfänglich angeordnete Vorsprünge aufweist, die beim Einbringen des Filtermoduls, insbesondere des rohrförmigen Filtermoduls, in den Aufnahmebereich der Verbindungseinrichtung in der Nut geführt werden. Die umfängliche Auflagefläche bildet dabei einen Bereich des seitlichen, insbesondere radialen Anschlusses, der einen kreisrunden Querschnitt aufweist. Das Einbringen des Filtermoduls, insbesondere des rohrförmigen Filtermoduls, wird somit erleichtert und ist sicherer gegen ein Verkanten des Filtermoduls im Aufnahmebereich der Verbindungseinrichtung.

In einer weiteren Ausführungsform ist die Anschlussvorrichtung so ausgebildet, dass der obere Vorsprung, auf dem die obere der beiden Führungsflächen des ersten Führungsteils verläuft, gegenüber dem unteren Vorsprung, auf dem die untere Führungsfläche verläuft, am proximalen Ende des Führungsteils verkürzt ausgearbeitet ist, so dass durch den unteren Vorsprung ein Absatz gebildet wird. In einer alternativen Ausführungsform sind die oberen Vorsprünge, auf denen die oberen Führungsflächen des ersten Führungsteils und des zweiten Führungsteils verlaufen, gegenüber den unteren Vorsprüngen, auf denen die unteren Führungsflächen verlaufen, am proximalen Ende verkürzt ausgearbeitet, so dass durch die unteren Vorsprünge Absätze gebildet werden. Das in den Aufnahmebereich der Verbindungseinrichtung einzubringende Filtermodul, insbesondere rohrförmige Filtermodul, kann so zunächst mit einer umfänglich angeordneten Auflagefläche oder mit mehreren umfänglich angeordneten Vorsprüngen auf den durch den unteren Vorsprung gebildeten Absatz abgesetzt werden. Die umfängliche Ablagefläche oder die mehreren umfänglich angeordneten Vorsprünge am rohrförmigen Filtermodul können so sicher in die Nut der Führungsteile eingeführt werden und der Prozess des Einbringens des Filtermoduls, insbesondere des rohrförmigen Filtermoduls, in den Aufnahmebereich der Verbindungseinrichtung sicherer und erleichtert werden.

In einer weiteren Ausführungsform ist die Anschlussvorrichtung dadurch gekennzeichnet, dass die Verbindungseinrichtung einen Verriegelungsmechanismus aufweist. Eine "*Verriegelungsvorrichtung*" im Sinne der vorliegenden Anmeldung bezeichnet dabei mechanische Sperrmittel, die ein Lösen des Filtermoduls, insbesondere des rohrförmigen Filtermoduls, aus dem Aufnahmebereich verhindern. Die Sperrung kann mechanisch formschlüssig oder kraftschlüssig erfolgen. In einer Ausführungsform wird die Verriegelung durch einen Stift bewirkt, der in eine Bohrung eingebracht wird, die durch die Führungsteile der Verbindungseinrichtung und durch den oder die umfänglichen Vorsprünge am rohrförmigen Filtermodul verläuft. Weiterhin können an den proximalen Enden der Führungsteile vorspannende Schnallen, Schnellspanner oder Klammern angebracht sein, die vom proximalen Ende des ersten Führungsteils zum proximalen Ende des zweiten Führungsteils übergreifen und im verriegelten Zustand das Filtermodul, insbesondere das rohrförmige Filtermodul, umgreifen.

In einer weiteren Ausführungsform ist die Anschlussvorrichtung dadurch gekennzeichnet, dass sie mindestens eine weitere Verbindungseinrichtung zur Aufnahme wenigstens eines koaxial zur Längsachse des rohrförmigen Filtermoduls angeordneten Anschlusses aufweist. Die Verbindungseinrichtung zur Aufnahme eines koaxialen Anschlusses am rohrförmigen Filtermodul weist ebenfalls ein Konnektorstück auf, das für die Konnektion des koaxialen Anschlusses vorgesehen ist. Das Konnektorstück weist ein inneres Lumen auf und kann aus einem Schlauchstück oder einer Hülse bestehen. Im Sinne der vorliegenden Anmeldung wird die Verbindungseinrichtung zur Aufnahme eines koaxialen Anschlusses am rohrförmigen Filtermodul auch als koaxiale Verbindungseinrichtung bezeichnet.

Das Konnektorstück der koaxialen Verbindungseinrichtung besteht aus einem Plastikmaterial oder einem Metall. Dabei kann das Konnektorstück so ausgearbeitet sein, dass das Lumen des Konnektorstücks zumindest in einem Endabschnitt gegenüber der äußeren Abmessung des koaxialen Anschlusses des Filtermoduls ein Übermaß aufweist, so dass eine Konnektion durch Einschieben des koaxialen Anschlusses in das koaxiale Konnektorstück möglich ist. In einer Alternative kann das Konnektorstück aber auch so ausgearbeitet sein, dass das Lumen des koaxialen Anschlusses des Filtermoduls gegenüber den äußeren Abmessungen des koaxialen Konnektorstücks ein Übermaß aufweist, so dass das Konnektorstück in das Ende des Anschlusses einschiebbar ist und eine Konnektion erfolgen kann. Eine Dichtung der Konnektion kann durch Dichtelemente ausgeführt werden, die im Bereich der Kontaktflächen von Konnektorstück und Anschluss eingebracht sind. Solche Dichtelemente sind z.B. O-Ringe, die umfänglich auf der äußeren Oberfläche oder auf der inneren Oberfläche, lumenseitig des Anschlusses des Filtermoduls aufgebracht sind. Alternativ oder zusätzlich können solche O-Ringe auch auf den Konnektorstücken auf der äußeren oder der inneren, lumenseitigen Oberfläche der Konnektorstücke aufgebracht sein.

Im Unterschied zu der Verbindungseinrichtung, die der Konnektion des seitlichen, insbesondere des radialen Anschlusses des rohrförmigen Filtermoduls dient, wird die Konnektion zwischen koaxialem Anschluss und koaxialem Konnektorstück nicht durch eine Verriegelungsvorrichtung gesperrt. Dies bedeutet, dass das koaxiale Konnektorstück und der koaxiale Anschluss im konnektierten Zustand gegeneinander beweglich sind. Insbesondere kann eine solche Beweglichkeit erforderlich sein, um Wärmeausdehnungen des rohrförmigen Filtermoduls in Längsausrichtung ohne Materialspannungen kompensieren zu können.

Ein zweiter Aspekt der vorliegenden Offenbarung betrifft ein Filtermodul, insbesondere ein rohrförmiges Filtermodul, das in eine Anschlussvorrichtung der vorab beschriebenen Art eingebracht werden kann. Das Filtermodul weist ein rohrförmiges Gehäuse auf, mit einem ersten Strömungsraum und einem zweiten Strömungsraum, die durch zumindest eine Membran voneinander getrennt sind, weiterhin wenigstens einem ersten und einem zweiten Anschluss, die an dem Filtermodul angebracht sind, wobei der zumindest erste Anschluss mit dem ersten Strömungsraum und der zumindest zweite Anschluss mit dem zweiten Strömungsraum in Fluidverbindung stehen, wobei der zumindest erste Anschluss seitlich, vorzugsweise radial am Filtermodul angebracht ist und wobei das Filtermodul in Höhe des einen ersten, seitlich angeordneten Anschlusses einen oder mehrere umfänglich angeordnete Vorsprünge aufweist, wobei die umfänglich angeordnete Auflagefläche (305) in Höhe der mittleren horizontalen Querschnittsfläche des seitlichen, insbesondere des radialen Anschlusses liegt und wobei die umfänglich angeordnete Auflagefläche so ausgebildet ist, dass sie in eine Nut (210) des zumindest ersten Führungsteils (202) der Anschlussvorrichtung (100), wie in Anspruch 4 definiert, eingreifen kann

Das Filtermodul weist den Vorteil auf, dass es über die umfänglich angeordnete Auflagefläche oder die mehreren umfänglich angeordneten Vorsprünge auf den Führungsteilen der Verbindungseinrichtung zur Konnektion des seitlichen, insbesondere des radialen Anschlusses, aufgesetzt werden kann, um das Filtermodul in die Verbindungseinrichtung einzubringen. Der umfängliche Vorsprung stellt damit eine Führungshilfe dar, um das rohrförmige Filtermodul zur Konnektion zu bringen. Vorzugsweise wird die umfängliche Auflagefläche oder die mehreren umfänglichen Vorsprünge dazu genutzt, um das Filtermodul in die wenigstens eine Nut, oder in beide Nuten der Führungsteile der Verbindungseinrichtung einzubringen. Die Nut in dem wenigstens einem Führungsteil oder in dem wenigstens ersten und zweiten Führungsteil wirkt zusammen mit der umfänglichen Auflagefläche oder den umfänglich angeordneten Vorsprüngen am Filtermodul, insbesondere am rohrförmigen Filtermodul, als verbesserte Führungshilfe, um das Filtermodul in den Aufnahmebereich der Verbindungseinrichtung einzubringen. In einer bevorzugten Ausführungsform ist die umfängliche Auflagefläche ringförmig oder Teil eines Rings.

Durch die umfänglich angeordnete Auflagefläche, die in Höhe der mittleren horizontalen Querschnittsfläche des seitlichen Anschlusses liegt kann die Verbindungseinrichtung konstruktiv vorteilhaft ausgestaltet werden.

In einer weiteren Ausführungsform weist das Filtermodul, insbesondere das rohrförmige Filtermodul, zumindest drei Anschlüsse auf, die an dem Filtermodul angeordnet sind, wobei der erste Anschluss mit dem ersten Strömungsraum in Fluidverbindung steht und der zweite Anschluss und der dritte Anschluss mit dem zweiten Strömungsraum in Fluidverbindung stehen.

In einer alternativen Ausführungsform stehen der erste Anschluss und der zweite Anschuss mit dem ersten Strömungsraum in Fluidverbindung und der dritte Anschluss steht mit dem zweiten Strömungsraum in Fluidverbindung.

In einer weiteren Ausführungsform ist der erste Anschluss seitlich, insbesondere radial, am Filtermodul, insbesondere am rohrförmigen Filtermodul, angeordnet, wobei der zweite und der dritte Anschluss koaxial zur Längsachse des rohrförmigen Filtermoduls angeordnet sind.

In einer weiteren Ausführung ist das Filtermodul, insbesondere das rohrförmige Filtermodul, dadurch gekennzeichnet, dass zumindest vier Anschlüsse an dem Filtermodul angeordnet sind, wobei der erste Anschluss und der zumindest vierte Anschluss mit dem ersten Strömungsraum und der zweite und der dritte Anschluss mit dem zweiten Strömungsraum in Fluidverbindung stehen. In dieser Anordnung der Anschlüsse sind der erste und der vierte Anschluss seitlich, insbesondere radial am Filtermodul angeordnet, wobei der zweite und der dritte Anschluss koaxial zur Längsachse des Filtermoduls angeordnet sind. Insbesondere ist in einer weiteren Ausführungsform vorgesehen, dass ein derart ausgestaltetes rohrförmiges Filtermodul eine weitere umfänglich angeordnete Auflagefläche oder mehrere weitere umfänglich angeordnete Vorsprünge im Bereich des vierten Anschlusses aufweist. Ein derart ausgestaltetes Filtermodul eignet sich auch als Dialysefilter.

In einem weiteren Aspekt betrifft die Erfindung ein Filtrationssystem bestehend aus einer Anschlussvorrichtung nach wenigstens einer Ausführung des ersten Aspekts der Erfindung und wenigstens einem Filtermodul, insbesondere einem rohrförmigen Filtermodul nach wenigstens einer Ausführungsform des zweiten Aspekts der Erfindung.

In einer Ausführungsform zeichnet sich das Filtrationssystem dadurch aus, dass das Filtermodul, insbesondere das rohrförmige Filtermodul, in der Anschlussvorrichtung über die radiale Verbindungseinrichtung und die Verriegelungseinrichtung im Anschlusssystem fixiert und eine Konnektion zum Filtermodul hergestellt ist. Die koaxialen Verbindungseinrichtungen stellen dabei ebenfalls eine Konnektion zum rohrförmigen Filtermodul her. Die koaxialen Verbindungseinrichtungen sind aber nicht am rohrförmigen Filtermodul fixiert, so dass die koaxialen Anschlüsse des Filtermoduls in der Konnektion in Richtung der Längsachse des Filtermoduls beweglich sind. Das System bietet somit den Vorteil, dass Wärmeausdehnungen in Längsrichtung des Filtermoduls durch die beweglichen koaxialen Konnektionen kompensiert werden können und somit nicht in der Konstruktion des Systems durch Materialspannungen kompensiert werden müssen.

Die Konnektorstücke der koaxialen Verbindungseinrichtungen können verschiebliche Hülsen umfassen, die in axialer Richtung zur Längsachse des rohrförmigen Filtermoduls verschoben werden können. Es kann vorgesehen sein, dass die verschieblichen Hülsen über Hebel bewegt werden. Die Hebel können bevorzugt die Hülsen in eine rotatorische Bewegung versetzen. Es ist bevorzugt, im Konnektorstück eine Steigungsnut vorzusehen, die eine rotatorische Bewegung der verschieblichen Hülse in eine translatorische Bewegung in axialer Richtung umwandeln kann. Eine solche Ausgestaltung gewährleistet die spannungsarme Aufnahme von Materialausdehnungen und gleichzeitig die besonders einfache Montage des Filtermoduls, insbesondere betreffend die koaxiale Konnektion.

In einem vierten Aspekt betrifft die Erfindung eine Baugruppe aus einer Vielzahl von Filtrationssystemen nach dem dritten Aspekt der Erfindung, die sich dadurch auszeichnet, dass die Filtrationssysteme mit Verbindungskanälen miteinander in Fluidverbindung stehen. Derartige Baugruppen eignen sich insbesondere für Wasseraufbereitungsanlagen. Der Vorteil der erfindungsgemäßen Baugruppe besteht darin, dass die Wärmeausdehnung der Filtermodule, insbesondere der rohrförmigen Filtermodule, nicht durch konstruktive Teile der Baugruppe durch Materialdehnung kompensiert werden muss. Insbesondere ergibt sich dadurch ein Sicherheitsvorteil, da die Materialdehnung zu Defekten in der Baugruppe, insbesondere zu Undichtigkeiten der Konnektion zwischen den Verbindungseinrichtungen und den Anschlüssen am Filtermodul führen kann.

Ein fünfter Aspekt der Erfindung betrifft die Verwendung eines Filtermoduls nach einer Ausführung des zweiten Aspekts der Erfindung in der Verarbeitung von Fluiden, insbesondere von Flüssigkeiten in Filtrationsprozessen, insbesondere in der Wasseraufbereitung.

Ein sechster Aspekt der Erfindung betrifft die Verwendung einer Anschlussvorrichtung nach dem ersten Aspekt der Erfindung in Prozessen der Verarbeitung von Fluiden, insbesondere von Flüssigkeiten in Filtrationsprozessen, insbesondere in der Wasseraufbereitung.

## BESCHREIBUNG DER ERFINDUNG ANHAND DER FIGUREN

Figur 1 zeigt eine perspektivische Explosionsansicht einer erfindungsgemäßen Anschlussvorrichtung mit einem Filtermodul, jeweils gemäß einem Ausführungsbeispiel der Erfindung.

Figur 2 zeigt, in einer perspektivischen Seitenansicht, eine Verbindungseinrichtung einer Anschlussvorrichtung gemäß einem Ausführungsbeispiel der Erfindung.

Figur 3 zeigt die Verbindungseinrichtung der Figur 2 in einer Aufsicht aus einer Richtung parallel der Längsrichtung des Filtermoduls, wenn dieses in der Aufnahmeposition angeordnet ist.

Figur 4a zeigt die Verbindungseinrichtung der Figur 2 in einer weiteren Aufsicht aus einer Richtung parallel der Längsrichtung des Filtermoduls, wie dieses in die Aufnahmeposition bewegt wird.

Figur 4b zeigt die Verbindungseinrichtung mit Filtermodul der Figur 4a in Aufsicht in der Aufnahmeposition.

Figur 4c zeigt schematisch die s-förmige Kontur der die Führungsfläche aufweisenden Seite des Führungsteils der Verbindungseinrichtung der Figuren 1 bis 4b.

Figur 5 zeigt in einer perspektivischen Seitenansicht eine erfindungsgemäße Baugruppe gemäß einem Ausführungsbeispiel, die eine Vielzahl von Filtrationssystemen aufweist, und die mindestens eine erfindungsgemäße Anschlussvorrichtung gemäß einem Ausführungsbeispiel sowie Filtermodule gemäß einem Ausführungsbeispiel aufweist.

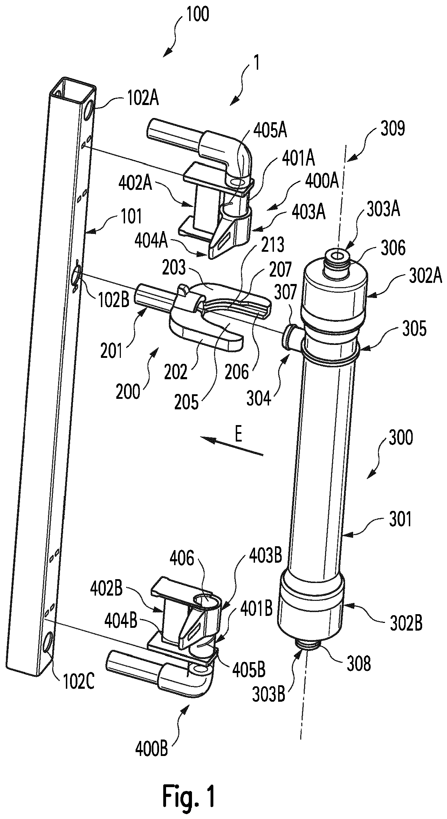

Figur 1 ist eine Darstellung einer erfindungsgemäßen Ausführungsform eines Filtrationssystems 1 in Form einer Explosionszeichnung. Das Filtrationssystem besteht aus einer Anschlussvorrichtung 100, einem rohrförmigen Filtermodul 300, einer Verbindungseinrichtung 200 zur Konnektion mit einem radialen Anschluss 304 des Filtermoduls 300 und zwei Verbindungseinrichtungen 400A, 400B zur Konnektion mit koaxialen Anschlüssen 303A, 303B des Filtermoduls. Die Verbindungseinrichtung 200 weist ein Konnektorstück 201 auf, durch das Fluide über den radialen Anschluss 304 des Filtermoduls 300 in einen Strömungsraum des Filtermoduls zu- oder abgeleitet werden können. Die Verbindungseinrichtungen 200, 400A, 400B sind dabei Teil der Anschlussvorrichtung 100. Die Anschlussvorrichtung 100 dient der Aufnahme und dem Anschluss des Filtermoduls 300, durch dessen Bewegung entlang der Richtung (E) aus einer proximalen Position, die durch den Anwender repräsentiert sein kann, in eine Aufnahmeposition, die ausgehend von der proximalen Position distal gelegen ist.

Weiterhin sind an der Verbindungseinrichtung 200 die Führungsteile 202, 203 gezeigt, die an dem Konnektorstück 201 der Verbindungseinrichtung 200 anschließen. Die Führungsteile weisen zum Aufnahmebereich 205 gerichtete Führungsflächen 207, 213 auf, von denen in Figur 1 nur die Führungsflächen am Führungsteil 203 zu sehen sind. Weiterhin zeigt Figur 1 ein rohrförmiges Filtermodul 300, das in den Aufnahmebereich 205 eingebracht werden kann, um den Anschluss 304 mit dem Konnektorstück 201 zu konnektieren und eine Fluidverbindung zwischen dem rohrförmigen Filtermodul 300 und der Anschlussvorrichtung 100 herzustellen. Das rohrförmige Filtermodul weist ein zylindrisches Gehäuse 301 auf, das von seinem äußeren Durchmesser so bemessen ist, dass es in den Aufnahmebereich 205 der Verbindungseinrichtung 200 aufgenommen werden kann. Das rohrförmige Filtermodul 300 weist weitere Anschlüsse 303A, 303B auf, die koaxial zur Längsachse 309 des rohrförmigen Filtermoduls 300 angeordnet sind. Die koaxialen Anschlüsse 303A, 303B sind dabei an den Enden des rohrförmigen Filtermoduls angeordnet und schließen an den Endkappen 302A, 302B des rohrförmigen Filtermoduls an. An dem radialen Anschluss 304 und den koaxial angeordneten Anschlüssen 303A, 303B sind O-Ringe 306, 307, 308 umfänglich auf die Anschlüsse aufgebracht, die zur Abdichtung der Anschlüsse gegenüber den Konnektorstücken 201, 401A, 401B der Verbindungseinrichtungen 200, 400A, 400B vorgesehen sind.

Die Anschlussvorrichtung 100 weist in der gezeigten Darstellung das Trägerteil 101 auf, das über Bohrungen 102A, 102B, 102C die Verbindungseinrichtungen 400A, 200, 400B aufnimmt. Durch Einbringen des Filtermoduls in die Verbindungseinrichtung 200, die den radialen Anschluss 302 konnektiert, und Anschließen der Verbindungseinrichtungen 400A, 400B, die die koaxialen Anschlüsse 303A, 303B konnektieren, liegen Anschlussvorrichtung 100 und rohrförmiges Filtermodul 300 zusammen als Filtrationssystem 1 vor. Die Fixierung des Filtermoduls in der Anschlussvorrichtung wird dabei über die Verbindungseinrichtung 200 am radialen Anschluss 304 des Filtermoduls und einer in der Figur nicht gezeigten Verriegelungseinrichtung vorgenommen. Die Verbindungseinrichtungen 400A, 400B, die die koaxialen Anschlüsse des Filtermoduls 300 konnektieren, sind dabei so ausgelegt, dass die Konnektion nicht fixiert wird.

Die Verbindungseinrichtungen 400A, 400B weisen die Konnektorstücke 401A, 401B zur Konnektion der koaxialen Anschlüsse 303A, 303B auf. Das Konnektorstück 401A kann auch als Schauglas ausgebildet sein bzw. ein Schauglas umfassen, um mit Hilfe eines Bubble-Tests eine Prüfung der Membranen auf Dichtigkeit zu ermöglichen. Die Konnektorstücke 401A, 401B umfassen weiterhin verschiebliche Hülsen 403A, 403B, die in axialer Richtung zur Längsachse verschoben werden können. In der gezeigten Ausführungsform können die verschieblichen Hülsen über Hebel 404A, 404B bewegt werden. Die Hebel 404A, 404B dienen dazu, die Hülsen 403a, 403B in rotatorische Bewegung zu versetzen. Über eine Steigungsnut 405A, 405B wird die rotatorische Bewegung in eine translatorische Bewegung umgewandelt, so dass die Hülse in axialer Richtung verschoben wird. Gemäß der in Figur 1 gezeigten Ausführungsform sind die koaxialen Anschlüsse 303A, 303B von ihrem Außendurchmesser so bemessen, dass sie in die Lumen 406 der verschieblichen Hülsen aufgenommen werden können. Das Lumen 406 der verschieblichen Hülsen 403A, 403B ist nur an einem der gezeigten Verbindungseinrichtungen 400B in der perspektivischen Darstellung der Fig. 1 sichtbar.

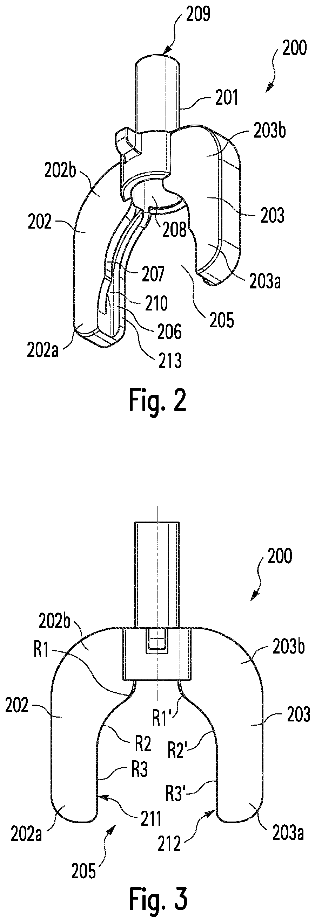

Fig. 2 zeigt eine perspektivische schematische Darstellung einer Ausführungsform der Verbindungseinrichtung 200, die zur Konnektion des radialen Anschlusses des Filtermoduls vorgesehen ist. Fig. 2 zeigt dabei eine detailliertere Darstellung der Verbindungseinrichtung 200 aus Figur 1. Die Verbindungseinrichtung 200 weist ein Konnektorstück 201 auf, das ein proximales Ende 208 mit einer Öffnung zum Lumen des Konnektorstücks und ein distales Ende 209 zeigt. Weiterhin sind die Führungsteile 202, 203 gezeigt, die am proximalen Ende des Konnektorstücks 208 anschließen. Die Führungsteile 202, 203 definieren in der Verbindungseinrichtung 200 einen Aufnahmebereich 205, der zur Aufnahme eines rohförmigen Filtermoduls vorgesehen ist. In der perspektivischen Darstellung der Figur 2 ist eine obere 207 und eine untere 213 Führungsfläche zu sehen. Die Führungsflächen sind so angeordnet, dass sie gegenüber der zwischen den Führungsflächen liegenden Nut 210 auf Vorsprüngen verlaufen. Die Nut 210 ist dabei dafür vorgesehen, die in Fig. 1 dargestellte umfängliche Auflagefläche 305 des Filtermoduls, insbesondere des rohrförmigen Filtermoduls 300, aufzunehmen, wenn das Filtermodul 300 in den Aufnahmebereich 205 der Verbindungseinrichtung 200 eingebracht wird. Die in der Darstellung in Fig. 2 gezeigten Führungsflächen 207, 213 sind so ausgestaltet, dass der Vorsprung, auf dem die obere Führungsfläche 207 verläuft, kürzer ausgestaltet ist als der Vorsprung, auf der die untere Führungsfläche 213 verläuft. Dadurch wird durch den Vorsprung, auf dem die untere Führungsfläche verläuft, ein Absatz 206 gebildet. Dieser Absatz dient dazu, das rohrförmige Filtermodul mit der umfänglichen Auflagefläche aufzusetzen, bevor das rohrförmige Filtermodul weiter in den Aufnahmebereich 205 der Verbindungseinrichtung eingebracht wird. Gleichzeitig dient der Absatz dazu, die umfängliche Auflagefläche 305 des Filtermoduls 300 in eine Stellung zu bringen, um die ringförmige Auflagefläche 305 in die Nut 210 einzuführen. Führungsflächen 207, 213 und Nut 210 sind dabei so an den Führungsteilen 202, 203 ausgestaltet, dass sie von einem proximalen Ende 202a, 203a der Führungsteile 202, 203 bis zu einem distalen Ende 202b, 203b der Führungsteile verlaufen. Insbesondere sind die Führungsflächen 213, 207 und die Nut 210 so angeordnet, dass sie mit den distalen Enden 202b, 203b der Führungsteile 202, 203 an das proximale Ende 208 des Konnektorstücks 201 anschließen.

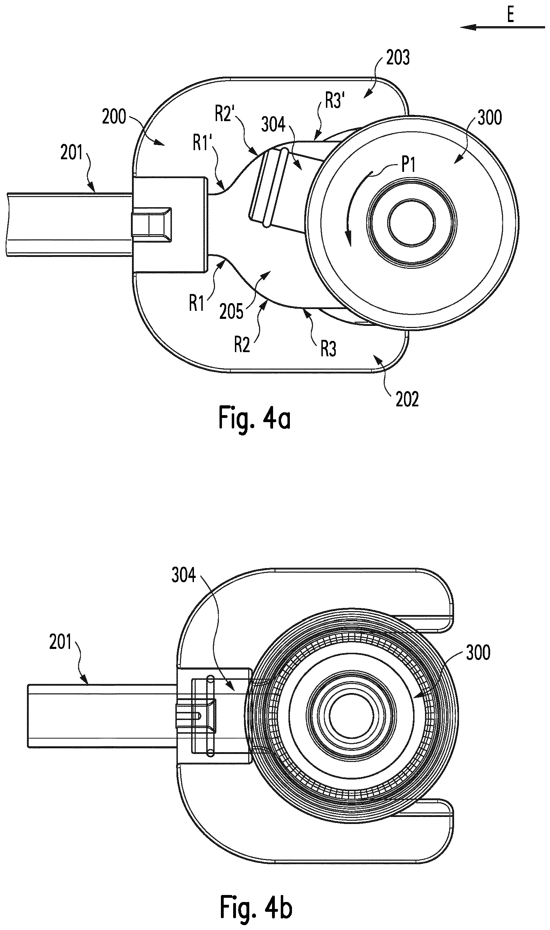

Fig. 3 zeigt eine Verbindungseinrichtung 200 in Aufsicht. Es ist jeweils die s-förmige, zum Aufnahmebereich 205 gerichtete Seite 211 der Führungsteile 202, 203 zu sehen. Die S-Form zeigt sich hier insbesondere einerseits in einem Querschnitt entlang der Richtung E - vorzugsweise senkrecht zur Längsrichtung eines Filtermoduls - durch die Führungsteile (siehe Figuren 4a, 4b), kann sich vorzugsweise, wie hier, aber auch in der Aufsicht aus einer Richtung parallel zur Längsrichtung eines Filtermoduls zeigen. Technisch wird genutzt, dass die vom Filtermodul beim Einschieben entlang der Richtung E kontaktierte Fläche die S-Form aufweist. Die S-Form entsteht durch zwei aneinanderreihende Krümmungsabschnitte (R1, R1', R2, R2') an den Führungsteilen 202, 203, die am distalen Ende 202b, 203b zum Aufnahmebereich 205 hin ausgerichtet sind. Ein dritter Abschnitt (R3, R3') bezeichnet einen geraden Abschnitt der Führungsteile am proximalen Ende 202a, 203a. Die lichte Weite im Aufnahmebereich 205 im Bereich der geraden Abschnitte (R3, R3') ist dabei im Wesentlichen gleich dem Außendurchmesser des Filtermoduls 300, das dafür vorgesehen ist, in den Aufnahmebereich eingebracht zu werden. Außendurchmesser des Filtermoduls 300 und lichte Weite des Aufnahmebereichs sind dabei so angepasst, dass das Filtermodul ohne Verkannten in den Aufnahmebereich eingebracht werden kann. Die lichte Weite des Aufnahmebereichs weist daher gegenüber dem Außendurchmesser des rohrförmigen Filtermoduls ein geringes Übermaß auf. Die Krümmungsabschnitte (R1, R1', R2, R2') können durch Krümmungsradien beschrieben werden. Die Krümmungsradien sind dabei so bemessen, dass sie den radialen Anschluss 304 des rohrförmigen Filtermoduls 300 beim Einbringen des Filtermoduls in den Aufnahmebereich 205 der Verbindungseinrichtung zum proximalen Ende 208 des Konnektorstücks ausrichten, so dass Konnektorstück 201 und radialer Anschluss 304 zur Konnektion gebracht werden können.

Figur 4a zeigt die Verbindungseinrichtung 200 der Figur 2 in einer weiteren Aufsicht aus einer Richtung parallel der Längsrichtung des Filtermoduls 300, wie dieses in die Aufnahmeposition aus einer proximalen Position bewegt wird. Es ist eine Position des Filtermoduls 300 gezeigt, in der eine nicht ausgerichtete Position des radialen Anschlusses 304 in dem Aufnahmebereich 205 vorliegt. Eine "nicht ausgerichtete Position" bedeutet dabei, dass der radiale Anschluss 304 nicht in Richtung zum Konnektorstück 201 der Verbindungseinrichtung 200 ausgerichtet und somit nicht konnektiertbar ist. Die Führungsflächen der Führungsteile sind in der Figur 4a nicht gezeigt. In der gezeigten Darstellung der Fig. 4a ist die zum Aufnahmebereich 205 gerichtete s-förmige Seite der Führungsteile 202, 203, auf der die Führungsflächen verlaufen, gezeigt. Weiterhin zeigt die Figur 4a kreissegmentförmige Krümmungsabschnitte (R1, R1', R2, R2'). Die kreissegmentförmigen Krümmungsabschnitte, der Durchmesser des Filtermoduls 300 und die lichte Weite des Aufnahmebereichs 205 im geraden Abschnitt (R3, R3') der Führungsteile 202, 203 sind so ausgelegt, dass der radiale Anschluss 304 beim Einbringen des Filtermoduls 300 in den Aufnahmebereich 205 an einer der in der Figur 4a nicht gezeigten seitlichen Führungsflächen der Führungsteile 202, 203 anliegt. Der radiale Anschluss des Filtermoduls 300 ist beim Einbringen in den Aufnahmebereich 205 immer nur mit einem der beiden Führungsteile 202, 203 in Kontakt. In der vorliegenden Figur 4a wird das Ausrichten des radialen Konnektors nur durch die kreissegmentförmigen Krümmungsabschnitte eines Führungsteils 203 unterstützt. Durch das weitere Einbringen des rohrförmigen Filtermoduls 300 in den Aufnahmebereich 205 der Verbindungseinrichtung 200 wird der radiale Anschluss 304 an den in Figur 4a nicht gezeigten Führungsflächen entlanggeführt und durch die kreissegmentförmigen Krümmungsabschnitte (R1', R2') des Führungsteils 203 ausgelenkt, so dass das Filtermodul im Aufnahmebereich eine Rotationsbewegung um seine Längsachse vollzieht. Die Drehrichtung der Rotation ist in Fig. 4a durch den Pfeil P1 angezeigt. Das rohrförmige Filtermodul wird dadurch in eine zweite Position gebracht, in der der radiale Anschluss 304 zum Konnektorstück ausgerichtet ist und eine Konnektion zwischen radialem Anschluss 304 und dem Konnektorstück 201 erfolgt.

Fig. 4b zeigt die Verbindungseinrichtung 200 mit Filtermodul 300 der Figur 4a in Aufsicht, in der Aufnahmeposition, in der sich das Filtermodul 300 in einer distalen Aufnahmeposition befindet und der Anschluss 304 in einer ausgerichteten Position vorliegt. In der "ausgerichteten Position" liegen radialer Anschluss 304 des Filtermoduls und Konnektorstück 201 in einem konnektierten Zustand vor. Fig. 4b zeigt dabei die Endposition des Filtermoduls 300 in der Verbindungseinrichtung 200, die das Filtermodul 300 durch weitere Drehung in Pfeilrichtung (P1) und Einschieben in den Aufnahmebereich 205 aus der nicht ausgerichteten Position von Fig. 4a einnimmt.

In den Figuren 4a/b ist der Radius des kreissegmentförmigen Krümmungsabschnitts R1 kleiner als der Radius des kreissegmentförmigen Krümmungsabschnitts R2. Der Radius des kreissegmentförmigen Krümmungsabschnitts R2 ist gleich groß oder größer als der Radius des rohrförmigen Filtermoduls. Insbesondere ist der Radius des kreissegmentförmigen Krümmungsabschnitts R1 weniger als halb so groß wie der Radius des Krümmungsabschnitts R2, oder in alternativen Ausführungsformen ist der Radius am Krümmungsabschnitt R1 um den Faktor 0,3 kleiner als der Radius am Krümmungsabschnitt R2. Durch diese besondere Gestaltung der Radienverhältnisse kann die gewünschte Drehbewegung optimal gestaltet werden.

In einer speziellen Ausführung betragen die Radien der kreissegmentförmigen Krümmungsabschnitte die folgenden Werte:

Radius am Abschnitt R1: 18,63 mm
Radius am Abschnitt R2: 57,15 mm
Außendurchmesser des rohrförmigen Filtermoduls: 57,15mm

Der Außendurchmesser des Filtermoduls und die lichte Weite im Abschnitt R3, R3' sind im Wesentlichen gleich groß. Es ist aber eine Spielpassung des Filtermoduls in dem Aufnahmebereich vorgesehen.

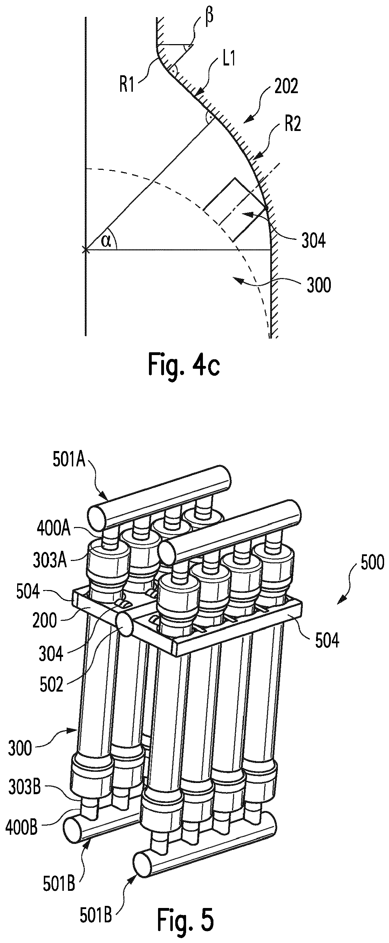

Fig. 4c zeigt in einer schematischen Querschnitt-Darstellung einen Teil eines Filtermoduls 300 in einer Verbindungseinrichtung 200 in einer nicht ausgerichteten Position. In Fig. 4c ist die s-förmige Kontur der Seite eines Führungsteils 202 gezeigt, die mit dem radialen Anschluss 304 des Filtermoduls in Kontakt steht. Die Führungsflächen des Führungsteils 202 sind in der Figur 4c nicht gezeigt. Die zum Aufnahmebereich 205 gerichtete Kontur des schematisch dargestellten Führungsteils 202 ist in der schematisch dargestellten Ausführungsform mit der Geometrie der Führungsflächen gleichzusetzen. In der gezeigten Darstellung der Fig. 4c wird weiterhin eine Ausführungsform der Verbindungseinrichtung gezeigt, in der die zum Aufnahmebereich gerichtete s-förmige Seite des gezeigten Führungsteils 202 einen linearen Abschnitt (L1) aufweist, der an dem ersten kreissegmentförmigen Krümmungsabschnitt (R1) und dem zweiten segmentförmigen Krümmungsabschnitt (R2) anschließt. Analog können auch die Führungsflächen beider Führungsteile einen linearen Abschnitt (L1) aufweisen. Gezeigt sind weiterhin die Winkel α und β der Kreissegmente in den Krümmungsabschnitten R1 und R2. Anhand der Darstellung aus Fig. 4c wird der Zusammenhang verdeutlicht, wonach für das Ausrichten des Filtermoduls 300 von einer nicht-ausgerichteten Position gemäß Fig. 4a in eine ausgerichtete Position gemäß Fig. 4b vorzugsweise folgende Bedingung eingehalten wird:

$$\tan \alpha < \frac{1}{\mu}$$

wobei:

α der Winkel des Kreissegmentes im R2 ist und
μ die Haftzahl des Materialpaares ist.

Für das Kreissegment R1 mit dem Winkel β gilt die analoge Beziehung. Das Materialpaar wird aus dem radialen Anschluss 304 und der in Fig. 4c nicht gezeigten Führungsfläche des Führungsteils gebildet. Vorzugsweise bestehen radialer Anschluss 304 und Führungsfläche aus Kunststoff, wie z.B. aus einem Polypropylenmaterial oder aus Polyvinylchlorid. Es können aber auch andere Materialien, wie beispielsweise Metalle, Verwendung finden. Für die jeweiligen Bauteile können gleiche oder unterschiedliche Materialien verwendet werden. Der Winkel des Kreissegmentes α und der lineare Abschnitt L1 kann zum reibungsarmen Einbringen des Filtermoduls in den Aufnahmebereich dementsprechend in Abhängigkeit von der Haftzahl μ angepasst werden.

Fig. 5 zeigt eine Ausführungsform einer erfindungsgemäßen Baugruppe 500, die aus einer Vielzahl von Filtrationssystemen besteht. Gezeigt sind rohrförmige Filtermodule 300, die in Verbindungseinrichtungen 200 zur Konnektion des radialen Anschlusses 304 eingebracht sind und die über weitere Verbindungseinrichtungen 400A und 400B an den koaxialen Anschlüssen 303A und 303B konnektiert sind. Die Verbindungseinrichtungen 200, 400A, 400B stellen eine Fluidverbindung mit Verbindungsrohren 501A, 501B, 502 her, mit denen Fluide zu den rohrförmigen Filtermodulen zugeleitet oder abgeleitet werden. Insbesondere können die Verbindungsrohre mit einem hydraulischen System einer Wasseraufbereitungsanlage verbunden sein. In der Baugruppe sind die rohrförmigen Filtermodule in Verbindungseinrichtungen am radialen Anschluss 304 über eine Verriegelungseinrichtung 504 fixiert. Die Verbindungseinrichtungen 400A, 400B fixieren die koaxialen Anschlüsse 303A, 303B in den Verbindungseinrichtungen dagegen nicht. Die koaxialen Anschlüsse sind daher bei thermischer Materialausdehnung in Längsrichtung der rohrförmigen Filtermodule in den Verbindungseinrichtungen 400A, 400B beweglich. Dadurch werden durch die thermische Materialausdehnung Materialspannungen auf die Verbindungsrohre 501A, 501B, 502 nur im nicht schädlichen Maße oder gar nicht übertragen.

## Patentansprüche

1. Anschlussvorrichtung (100) zur Aufnahme mindestens eines Filtermoduls (300), das durch Bewegung entlang einer Richtung (E) aus einer proximalen Position in eine distale Aufnahmeposition an die Anschlussvorrichtung anschließbar ist, insbesondere eines rohrförmigen Filtermoduls, mit wenigstens einem seitlichen Anschluss, insbesondere einem radialen (304) Anschluss, aufweisend

zumindest eine erste Verbindungseinrichtung (200)

mit einem ein inneres Lumen aufweisenden Konnektorstück (201), welches ein in der Aufnahmeposition zum Filtermodul weisendes proximales Ende (208) und ein zur Anschlussvorrichtung weisendes distales Ende (209) aufweist, wobei das proximale Ende (208) des Konnektorstücks (201) ausgebildet ist, eine Konnektion des Konnektorstücks (201) an dem seitlichen, insbesondere an dem radialen Anschluss (304) des Filtermoduls zu ermöglichen, und

mit zumindest einem ersten und einem zweiten Führungsteil (202, 203) mit jeweils einem proximalen Ende (202a, 203a) und einem distalen Ende (202b, 203b), die einen Aufnahmebereich (205) der Verbindungseinrichtung (200) zur Aufnahme eines Filtermoduls, insbesondere eines rohrförmigen Filtermoduls (300) definieren, wobei zumindest das erste Führungsteil (202) eine zu dem Aufnahmebereich (205) gerichtete Seite (211) mit zumindest einer Führungsfläche aufweist, die vom proximalen Ende (202a) zum distalen Ende (202b) des ersten Führungsteils (202) verläuft und am proximalen Ende (208) des Konnektorstücks (201) anschließt,

**dadurch gekennzeichnet, dass**
die zum Aufnahmebereich (205) gerichtete, die Führungsfläche aufweisende Seite (211) des Führungsteils (202) in einem Querschnitt s-förmig ausgestaltet ist, wobei die S-Form durch zwei aneinanderreihende Krümmungsabschnitte (R1, R2) entsteht und der eine Querschnitt dabei mit einer Fläche zusammenfällt, die durch den Aufnahmebereich (205) und die beiden Führungsteile (202, 203) der Verbindungseinrichtung verläuft.

2. Anschlussvorrichtung (100) nach Anspruch 1, **dadurch gekennzeichnet, dass** das zweite Führungsteil (203) eine zu dem Aufnahmebereich (205) der Verbindungseinrichtung gerichtete Seite (212) mit zumindest einer Führungsfläche aufweist, die vom proximalen Ende (203a) zum distalen Ende (203b) des zweiten Führungsteils (203) verläuft und am proximalen Ende (208) des Konnektorstücks (201) anschließt, wobei die zum Aufnahmebereich (205) gerichtete, die Führungsfläche aufweisende Seite (212) des Führungsteils (203) in einem Querschnitt s-förmig (R1, R2) ausgestaltet ist, wobei die S-Form durch zwei aneinanderreihende Krümmungsabschnitte (R1', R2') entsteht und der eine Querschnitt dabei mit einer Fläche zusammenfällt, die durch den Aufnahmebereich (205) und die beiden Führungsteile (202, 203) der Verbindungseinrichtung verläuft.

3. Anschlussvorrichtung (100) nach Anspruch 1 oder 2, **dadurch gekennzeichnet, dass** das erste Führungsteil (202) und das zweite Führungsteil (203) auskragend am Konnektorstück, vorzugsweise am proximalen Ende (208) des Konnektorstücks (201), angebracht sind.

4. Anschlussvorrichtung (100) nach wenigstens einem der vorangehenden Ansprüche, **dadurch gekennzeichnet, dass** die zu dem Aufnahmebereich (205) der Verbindungseinrichtung (200) gerichtete s-förmige Seite (211) des zumindest ersten Führungsteils (202) oder die zu dem Aufnahmebereich (205) des zumindest ersten Führungsteils (202) und des zweiten Führungsteils (203) gerichteten s-förmigen Seiten (211, 212) obere und untere Führungsflächen (207, 213) aufweisen, und eine dazwischen, entlang der Führungsflächen verlaufende Nut (210) angeordnet ist, so dass die Führungsflächen (207, 213) relativ zur Nut auf oberen und unteren Vorsprüngen verlaufen.

5. Anschlussvorrichtung (100) nach Anspruch 3 **dadurch gekennzeichnet, dass** der obere Vorsprung, auf dem die obere der beiden Führungsflächen (207, 213) des ersten Führungsteils (202) verläuft, gegenüber dem unteren Vorsprung, auf dem die untere Führungsfläche verläuft, am proximalen Ende des Führungsteils verkürzt ausgearbeitet ist, so dass durch den unteren Vorsprung ein Absatz (206) gebildet wird, oder **dadurch gekennzeichnet, dass** die oberen Vorsprünge, auf denen die oberen der beiden Führungsflächen des ersten Führungsteils (202) und des zweiten Führungsteils verlaufen, gegenüber den unteren Vorsprüngen, auf denen die unteren Führungsflächen verlaufen, am proximalen Ende (202a, 203a) der Führungsteile (202, 203) verkürzt ausgearbeitet sind, so dass durch die unteren Vorsprünge Absätze gebildet werden.

6. Anschlussvorrichtung (100) nach wenigstens einem der vorangehenden Ansprüche, **dadurch gekennzeichnet, dass** die Anschlussvorrichtung (100), insbesondere die Verbindungseinrichtung (200), eine Verriegelungsvorrichtung aufweist, um das Filtermodul in dem Aufnahmebereich (205) der Verbindungseinrichtung (200) zu fixieren.

7. Anschlussvorrichtung (100) nach wenigstens einem der vorangehenden Ansprüche, **dadurch gekennzeichnet, dass** die Vorrichtung zumindest eine weitere Verbindungseinrichtung (400A, 400B) zur Aufnahme wenigstens eines koaxial zur Längsachse (309) des rohrförmigen Filtermoduls (300) angeordneten Anschlusses (303A, 303B) aufweist, wobei

optional die Verbindungseinrichtung (400A, 400B) zur Aufnahme wenigstens eines koaxial am Filter angeordneten Anschlusses (303A, 303B) eine bewegliche Lagerung des Anschlusses (303A, 303B) in der Verbindungseinrichtung (400A, 400B) ermöglicht.

**8.** Filtrationssystem (1) aufweisend zumindest eine Anschlussvorrichtung (100) nach wenigstens einem der Ansprüche 1 bis 7 und wenigstens ein Filtermodul (300) aufweisend ein rohrförmiges Gehäuse (301), einen ersten Strömungsraum und einen zweiten Strömungsraum, die durch zumindest eine Membran voneinander getrennt sind, zumindest einem ersten (304) und zumindest einem zweiten Anschluss (303A, 303B), die an dem Filtermodul angebracht sind, wobei der zumindest eine erste Anschluss (304) mit dem ersten Strömungsraum und der zumindest eine zweite Anschluss (303A, 303B) mit dem zweiten Strömungsraum in Fluidverbindung steht, wobei der eine erste Anschluss (304) seitlich, vorzugsweise radial am Filtermodul angebracht ist und das Filtermodul (300) in Höhe des ersten seitlich, angeordneten Anschlusses (304) eine umfänglich angeordnete Auflagefläche (305) oder mehrere umfänglich angeordnete Vorsprünge aufweist.

**9.** Baugruppe (500) aufweisend eine Mehrzahl von Filtrationssystemen (1) nach Anspruch 8 und zwischen den Filtrationssystemen angeordnete Verbindungskanäle (501A, 501B, 502).

**10.** Verwendung einer Anschlussvorrichtung nach wenigstens einem der Ansprüche 1 bis 7 in Prozessen zur Verarbeitung von Fluiden, insbesondere zur Verarbeitung von Flüssigkeiten in Filtrationsprozessen, insbesondere in der Wasseraufbereitung.

**Claims**

**1.** A connection device (100) for receiving at least one filter module (300) which is connectable to the connection device by moving from a proximal position into a distal receiving position along a direction (E), in particular a tubular filter module, having at least one lateral connector, in particular a radial connector (304), comprising

at least one first connecting apparatus (200) having a connector piece (201) with an inner lumen exhibiting a proximal end (208) facing the filter module and a distal end (209) facing the connection device in the receiving position, wherein the proximal end (208) of the connector piece (201) is designed to enable a connection of the connector piece (201) to the lateral, in particular radial connector (304) of the filter module, and
having at least one first and one second guide member (202, 203) exhibiting a respective proximal end (202a, 203a) and distal end (202b, 203b) which define a receiving area (205) of the connecting apparatus (200) for receiving a filter module, in particular a tubular filter module (300), wherein at least the first guide member (202) exhibits a side (211) directed toward the receiving area (205) which has at least one guide surface running from the proximal end (202a) to the distal end (202b) of the first guide member (202) and connecting to the proximal end (208) of the connector piece (201), **characterized in that** the side (211) of the guide member (202) facing the guide surface directed toward the receiving area (205) is in a cross section S-shaped, wherein the S-shape results from two sequentially assembled curved sections (R1, R2) and the cross section coincides with an area that extends through the receiving area (205) and both of the guide members (202, 203) of the connecting apparatus.

**2.** The connection device (100) according to claim 1, **characterized in that** the second guide member (203) exhibits a side (212) directed toward the receiving area (205) of the connecting apparatus which has at least one guide surface running from the proximal end (203a) to the distal end (203b) of the second guide member (203) and connecting to the proximal end (208) of the connector piece (201), wherein the side (212) of the guide member (203) comprising the guide surface directed toward the receiving area (205) is in a cross section S-shaped, wherein the S-shape results from two sequentially assembled curved sections (R1, R2) and the cross section coincides with an area that extends through the receiving area (205) and both of the guide members (202, 203) of the connecting apparatus.

**3.** The connection device (100) according to claim 1 or 2, **characterized in that** the first guide member (202) and the second guide member (203) are cantilevered to the connector piece, preferably at the proximal end (208) of the connector piece (201).

**4.** The connection device (100) according to at least one of the preceding claims, **characterized in that** the S-shaped side (211) of the at least one guide member (202) directed toward the receiving area (205) of the connecting apparatus (200) or the S-shaped sides (211, 212) directed toward the receiving area (205) of the at least first guide member (202) and the second guide member (203) comprise upper and lower guide surfaces (207, 213) and a groove (210) extending along the guide surfaces between same is arranged such that the guide surfaces (207, 213) run on upper and lower projections relative to the groove.

5. The connection device (100) according to claim 3, **characterized in that** the upper projection on which the upper of the two guide surfaces (207, 213) of the first guide member (202) runs is of shorter configuration at the proximal end of the guide member compared to the lower projection on which the lower guide surface runs such that the lower projection forms a platform (206), or **characterized in that** the upper projections on which the upper of the two guide surfaces of the first guide member (202) and the second guide member run are of shorter configuration at the proximal ends (202a, 203a) of the guide members (202, 203) compared to the lower projections on which the lower guide surfaces run such that the lower projections form platforms.

6. The connection device (100) according to at least one of the preceding claims, **characterized in that** the connection device (100), in particular the connecting apparatus (200), comprises a locking mechanism in order to fix the filter module in the receiving area (205) of the connecting apparatus (200).

7. The connection device (100) according to at least one of the preceding claims, **characterized in that** the apparatus comprises at least one further connecting apparatus (400A, 400B) for receiving at least one connector (303A, 303B) arranged coaxially to the longitudinal axis (309) of the tubular filter module (300), wherein
optionally the connecting apparatus (400A, 400B) for receiving at least one connector (303A, 303B) arranged coaxially on the filter enables a movable supporting of the connector (303A, 303B) in the connecting apparatus (400A, 400B).

8. A filtration system (1) comprising at least one connection device (100) according to at least one of claims 1 to 7 and at least one filter module (300) comprising a tubular housing (301), a first flow chamber and a second flow chamber which are separated from each other by at least one membrane, at least one first (304) and at least one second connector (303A, 303B) attached to the filter module, wherein the at least one first connector (304) is in fluid communication with the first flow chamber and the at least one second connector (303A, 303B) is in fluid communication with the second flow chamber, wherein the one first connector (304) is laterally, preferably radially, fixed on the filter module the filter module (300) comprises a circumferentially arranged bearing surface (305) or a plurality of circumferentially arranged projections at the height of the first laterally arranged connector (304).

9. An assembly (500) comprising a plurality of filtration systems (1) in accordance with claim 8 and connecting channels (501A, 501B, 502) arranged between said filtration systems.

10. The use of a filter module (300) in accordance with at least one of claims 1 to 7 in processes of fluid processing, in particular for processing liquids in filtration processes, particularly in water treatment.

**Revendications**

1. Dispositif de raccord (100) destiné à recevoir au moins un module de filtre (300) qui peut être raccordé au dispositif de raccord par déplacement dans une direction (E) depuis une position proximale jusque dans une position de réception distale, en particulier un module de filtre tubulaire, avec au moins un raccord latéral, en particulier un raccord radial (304), présentant

au moins un premier équipement de liaison (200)
avec une pièce de connecteur (201) présentant une lumière intérieure, laquelle pièce de connecteur présente une extrémité proximale (208) pointant dans la position de réception vers le module de filtre et une extrémité distale (209) pointant vers le dispositif de raccord, dans lequel l'extrémité proximale (208) de la pièce de connecteur (201) est conçue pour permettre une connexion de la pièce de connecteur (201) avec le raccord latéral, en particulier radial (304) du module de filtre, et
avec au moins une première et une seconde partie de guidage (202, 203) avec respectivement une extrémité proximale (202a, 203a) et une extrémité distale (202b, 203b) qui définissent une zone de réception (205) de l'équipement de liaison (200) pour la réception d'un module de filtre, en particulier d'un module de filtre tubulaire (300), dans lequel au moins la première partie de guidage (202) présente un côté (211) orienté vers la zone de réception (205) avec au moins une surface de guidage qui se déroule depuis l'extrémité proximale (202a) vers l'extrémité distale (202b) de la première partie de guidage (202) et se raccorde à l'extrémité proximale (208) de la pièce de connecteur (201), **caractérisé en ce que**
le côté (211), présentant la surface de guidage et orienté vers la zone de réception (205), de la partie de guidage (202) est conçu en forme de S dans une coupe transversale, dans lequel la forme en S est obtenue par deux sections de courbure (R1, R2) en série l'une à la suite de l'autre et ce faisant la une coupe transversale coïncide avec une surface qui se déroule à travers la zone de réception (205) et les deux parties de guidage (202, 203) de l'équipement de liaison.

2. Dispositif de raccord (100) selon la revendication 1, **caractérisé en ce que** la seconde partie de guidage (203) présente un côté (212) orienté vers la zone de réception (205) de l'équipement de liaison avec au moins une surface de guidage qui se déroule depuis l'extrémité proximale (203a) vers l'extrémité distale (203b) de la seconde partie de guidage (203) et se raccorde à l'extrémité proximale (208) de la pièce de connecteur (201), dans lequel le côté (212), présentant la surface de guidage et orienté vers la zone de réception (205), de la partie de guidage (203) est conçu en forme de S (R1, R2) dans une coupe transversale, dans lequel la forme en S est obtenue par deux sections de courbure (R1', R2') en série l'une à la suite de l'autre et ce faisant, la une coupe transversale coïncide avec une surface qui se déroule à travers la zone de réception (205) et les deux parties de guidage (202, 203) de l'équipement de liaison.

3. Dispositif de raccord (100) selon la revendication 1 ou 2, **caractérisé en ce que** la première partie de guidage (202) et la seconde partie de guidage (203) sont placées en faisant saillie au niveau de la pièce de connecteur, de préférence au niveau de l'extrémité proximale (208) de la pièce de connecteur (201).

4. Dispositif de raccord (100) selon au moins l'une des revendications précédentes, **caractérisé en ce que** le côté (211), en forme de S et orienté vers la zone de réception (205) de l'équipement de liaison (200), de la au moins première partie de guidage (202) ou les côtés (211, 212), en forme de S et orientés vers la zone de réception (205) de la au moins première partie de guidage (202) et de la seconde partie de guidage (203), présentent des surfaces de guidage (207, 213) supérieure et inférieure et une rainure (210) agencée entre celles-ci et se déroulant le long des surfaces de guidage, de sorte que les surfaces de guidage (207, 213) se déroulent sur des saillies supérieure et inférieure par rapport à la rainure.

5. Dispositif de raccord (100) selon la revendication 3, **caractérisé en ce que** la saillie supérieure sur laquelle se déroule la surface de guidage supérieure parmi les deux surfaces de guidage (207, 213) de la première partie de guidage (202) est travaillée de façon à être raccourcie au niveau de l'extrémité proximale de la partie de guidage, par rapport à la saillie inférieure sur laquelle se déroule la surface de guidage inférieure, de sorte qu'un épaulement (206) soit formé par la saillie inférieure, ou **caractérisé en ce que** les saillies supérieures sur lesquelles se déroulent les surfaces de guidage supérieures parmi les deux surfaces de guidage de la première partie de guidage (202) et de la seconde partie de guidage sont travaillées de façon à être raccourcies au niveau de l'extrémité proximale (202a, 203a) des parties de guidage (202, 203), par rapport aux saillies inférieures sur lesquelles se déroulent les surfaces de guidage inférieures, de sorte que des épaulements soient formés par les saillies inférieures.

6. Dispositif de raccord (100) selon au moins l'une des revendications précédentes, **caractérisé en ce que** le dispositif de raccord (100), en particulier l'équipement de liaison (200), présente un dispositif de verrouillage pour fixer le module de filtre dans la zone de réception (205) de l'équipement de liaison (200).

7. Dispositif de raccord (100) selon au moins l'une des revendications précédentes, **caractérisé en ce que** le dispositif présente au moins un autre équipement de liaison (400A, 400B) pour la réception d'au moins un raccord (303A, 303B) agencé de façon coaxiale par rapport à l'axe longitudinal (309) du module de filtre (300) tubulaire, dans lequel optionnellement l'équipement de liaison (400A, 400B) permet une installation mobile du raccord (303A, 303B) dans l'équipement de liaison (400A, 400B), pour la réception d'au moins un raccord (303A, 303B) agencé de façon coaxiale par rapport au filtre.

8. Système de filtration (1) présentant au moins un dispositif de raccord (100) selon au moins l'une des revendications 1 à 7 et au moins un module de filtre (300) présentant un logement (301) tubulaire, un premier espace d'écoulement et un second espace d'écoulement qui sont séparés l'un de l'autre par au moins une membrane, au moins un premier (304) et au moins un second raccord (303A, 303B) qui sont placés contre le module de filtre, dans lequel le au moins un premier raccord (304) est en communication fluidique avec le premier espace d'écoulement et le au moins un second raccord (303A, 303B) est en communication fluidique avec le second espace d'écoulement, dans lequel le un premier raccord (304) est placé latéralement, de préférence radialement, contre le module de filtre et le module de filtre (300) présente, à hauteur du premier raccord (304) agencé latéralement, une surface d'appui (305) agencée de façon circonférentielle ou plusieurs saillies agencées de façon circonférentielle.

9. Assemblage (500) présentant une pluralité de systèmes de filtration (1) selon la revendication 8 et des canaux de liaison (501A, 501B, 502) agencés entre les systèmes de filtration.

**10.** Utilisation d'un dispositif de raccord selon au moins l'une des revendications 1 à 7 dans des processus destinés à traiter des fluides, en particulier destinés à traiter des liquides dans des processus de filtration, en particulier dans le traitement de l'eau.

Fig. 1

Fig. 2

Fig. 3

E

203

200

201

R2'

R1'

304

R3'

300

P1

R1

205

R2

R3

202

Fig. 4a

304

201

300

Fig. 4b

Fig. 4c

Fig. 5

21

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2012113505 A1
- DE 19727251 A1
- US 20150001140 A
- JP 2000325712 A